# EUROPEAN PATENT APPLICATION

(11) **EP 2 428 242 A1**
(43) Date of publication of application: **14.03.2012**
(21) Application number: 11180642.8
(22) Date of filing: 08.09.2011
(51) Int. Cl.: A61M 16/01, A61M 16/10

(54) **Anesthesia system and method**

(30) Priority: 13.09.2010 US 880297
(71) Applicant: General Electric Company, Schenectady, NY 12345 (US)
(72) Inventor: Mashak, James Nyal, Madison, Wisconsin 53718 (US)
(74) Representative: Illingworth-Law, William Illingworth

(57) **Abstract**

A system is disclosed herein. The system may include a pneumatic circuit (30) comprising an inspiratory limb (40), an expiratory limb (42), and an endotracheal tube (45). The endotracheal tube (45) may be configured to form a pneumatic coupling between a patient (34), the inspiratory limb (40) and the expiratory limb (42). The system may be configured to automatically identify the presence of a leak in the pneumatic coupling.

## Description

### FIELD OF THE INVENTION

This disclosure relates generally to a system and method configured to automatically identify improper endotracheal tube placement.

### BACKGROUND OF THE INVENTION

In general, medical ventilators systems are used to provide respiratory support to patients undergoing anesthesia and respiratory treatment whenever the patient's ability to breath is compromised. The primary function of the medical ventilator system is to maintain suitable pressure and flow of gases inspired and expired by the patient. Gases may be transferred to and from the patient via an endotracheal tube. The process of placing an endotracheal tube into a patient's trachea is called tracheal intubation.

One problem with conventional medical ventilator systems relates to the performance of the tracheal intubations. More precisely, improper placement of an endotracheal during a tracheal intubation can produce a pneumatic leak. A pneumatic leak can dislodge the endotracheal tube, and interferes with the efficient transfer of breathing gases to and from the patient.

### BRIEF DESCRIPTION OF THE INVENTION

The above-mentioned shortcomings, disadvantages and problems are addressed herein which will be understood by reading and understanding the following specification.

In an embodiment, a system includes a pneumatic circuit comprising an inspiratory limb, an expiratory limb, and an endotracheal tube. The endotracheal tube is configured to form a pneumatic coupling between a patient, the inspiratory limb and the expiratory limb. The system also includes controller connected to the pneumatic circuit. The controller is configured to identify the presence of a leak in the pneumatic coupling.

In another embodiment, an anesthesia system includes a pneumatic circuit comprising an inspiratory limb, an expiratory limb, and an endotracheal tube. The endotracheal tube is configured to form a pneumatic coupling between a patient, the inspiratory limb and the expiratory limb. The pneumatic circuit also includes a first sensor in pneumatic communication with the inspiratory limb, and a second sensor in pneumatic communication with the expiratory limb. The anesthesia system also includes an anesthesia machine connected to the pneumatic circuit. The anesthesia machine comprises a controller connected to the first and second sensors. The controller is configured to transfer a gas into the inspiratory limb of the pneumatic circuit, and to identify the presence of a leak in the pneumatic coupling attributable to an improperly placed endotracheal tube based on feedback from the first and second sensors.

In another embodiment, a method includes transferring a gas into a pneumatic circuit, measuring an inspiratory flow rate of the gas through an inspiratory limb of the pneumatic circuit, and measuring an expiratory flow rate of the gas through an expiratory limb of the pneumatic circuit. The method also includes implementing a controller to automatically identify the presence of a pneumatic leak attributable to an improperly placed endotracheal tube based on the inspiratory flow rate and the expiratory flow rate.

Various other features, objects, and advantages of the invention will be made apparent to those skilled in the art from the accompanying drawings and detailed description thereof.

### BRIEF DESCRIPTION OF THE DRAWINGS

FIGURE 1 is a schematic representation of an anesthesia system in accordance with an embodiment;

FIGURE 2 is a schematic representation of a pneumatic circuit of the anesthesia system of Figure 1 in accordance with an embodiment; and

FIGURE 3 is a flow chart illustrating a method in accordance with an embodiment.

### DETAILED DESCRIPTION OF THE INVENTION

In the following detailed description, reference is made to the accompanying drawings that form a part hereof, and in which is shown by way of illustration specific embodiments that may be practiced. These embodiments are described in sufficient detail to enable those skilled in the art to practice the embodiments, and it is to be understood that other embodiments may be utilized and that logical, mechanical, electrical and other changes may be made without departing from the scope of the embodiments. The following detailed description is, therefore, not to be taken as limiting the scope of the invention.

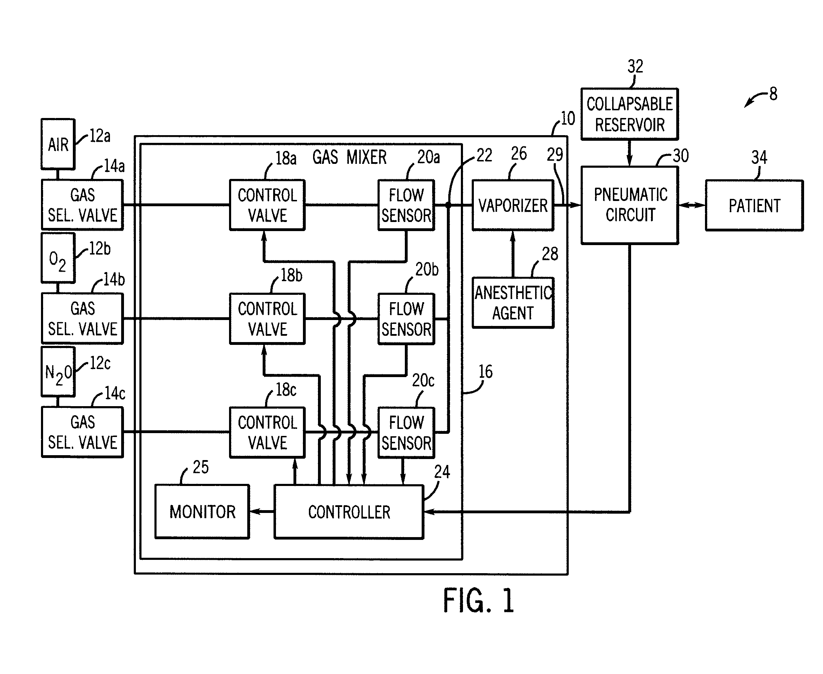

Referring to Figure 1, an anesthesia system 8 is schematically depicted in accordance with an embodiment. The anesthesia system 8 includes an anesthesia machine 10, a plurality of gas storage devices 12a, 12b and 12c, a plurality of gas selector valves 14a, 14b, and 14c, a pneumatic circuit 30, and a collapsible reservoir or breathing bag 32. The anesthesia machine 10 is shown for illustrative purposes and it should be appreciated that other types of anesthesia machines may alternately be implemented. In a typical hospital environment, the gas storage devices 12a, 12b and 12c are centrally located storage tanks configured to supply medical gas to multiple anesthesia machines and multiple hospital rooms. The storage tanks are generally pressurized to facilitate the transfer of the medical gas to the anesthesia machine 10.

The gas storage devices 12a, 12b and 12c will hereinafter be described as including an air tank 12a, an oxygen (02) tank 12b, and a nitrous oxide (N20) tank 12c, respectively, however it should be appreciated that other storage devices and other types of gas may alternatively be implemented. The gas storage tanks 12a, 12b and 12c are each connected to one of the gas selector valves 14a, 14b, and 14c, respectively. The gas selector valves 14a, 14b and 14c may be implemented to shut off the flow of medical gas from the storage tanks 12a, 12b and 12c when the anesthesia machine 10 is not operational. When one of the gas selector valves 14a, 14b and 14c is opened, gas from a respective storage tank 12a, 12b and 12c is transferred under pressure to the anesthesia machine 10.

The anesthesia machine 10 includes a gas mixer 16 adapted to receive medical gas from the storage tanks 12a, 12b and 12c. The gas mixer 16 includes a plurality of control valves 18a, 18b and 18c that are respectively connected to one of the gas selector valves 14a, 14b and 14c. The gas mixer 16 also includes a plurality of flow sensors 20a, 20b and 20c that are each disposed downstream from a respective control valve 18a, 18b, and 18c. After passing through one of the control valves 18a, 18b and 18c, and passing by one of the flow sensors 20a, 20b and 20c, the individual gasses (i.e., air, 02 and N20) are combined to form a mixed gas at the mixed gas outlet 22.

The control valves 18a, 18b and 18c and the flow sensors 20a, 20b and 20c are each connected to a controller 24. The controller 24 is configured to operate the control valves 18a, 18b and 18c in a response to gas flow rate feedback from the sensors 20a, 20b and 20c. Accordingly, the controller 24 can be implemented to maintain a selectable flow rate for each gas (i.e., air, 02 and N2O) such that the mixed gas at the mixed gas outlet 22 comprises a selectable ratio of air, 02 and N20. The mixed gas flows to a vaporizer 26 where an anesthetic agent 28 may be vaporized and added to the mixed gas from the mixed gas outlet 22. The anesthetic agent 28 and/or mixed gas combination is referred to as inhalation gas or fresh gas 29, which passes through the pneumatic circuit 30 and is delivered to the patient 34.

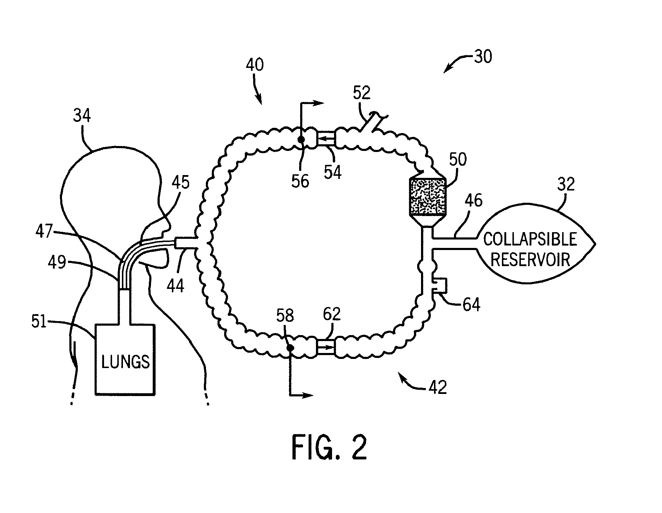

The pneumatic circuit 30 is configured to facilitate the transfer of fresh gas 29 from the anesthesia machine 10 to the patient 34, and to vent exhalation gas from the patient 34 to a hospital scavenging system (not shown). The pneumatic circuit 30 is also configured to generate a feedback signal from one or more of the sensors 56 and/or 58 (shown in Figure 2) that is transmittable to the controller 24. The collapsible reservoir 32 may be manually compressed to transfer fresh gas 29 to the patient 34 in a known manner.

Referring to Figure 2, an exemplary embodiment of the pneumatic circuit 30 is shown in more detail. The pneumatic circuit 30 may include an inspiratory channel or limb 40, an expiratory channel or limb 42, a Y-piece 44, an endotracheal tube 45, and a T-piece 46. The endotracheal tube 45 pneumatically couples the patient 34 with the Y-piece 44, the inspiratory limb 40 and the expiratory limb 42. The T-piece 46 pneumatically couples the collapsible reservoir 32 with the inspiratory limb 40 and the expiratory limb 42.

The inspiratory limb 40 comprises one or more tubes configured to direct fresh gas 29 and/or recycled exhalation gas to the patient 34. The inspiratory limb 40 may include a CO2 absorber 50, a fresh gas inlet 52, a one-way valve 54, and a flow sensor 56.

The CO2 absorber 50 is adapted to remove CO2 from the patient's exhalation gas to produce recycled exhalation gas. The recycled exhalation gas is transferable back to the patient 34 to reuse and thereby conserve anesthetic agent 28. The fresh gas inlet 52 is pneumatically coupled with and adapted to receive fresh gas 29 from the anesthesia machine 10. The one-way valve 54 is adapted to regulate fluid flow through the inspiratory limb 40 such that fluid is only transferable in a direction toward the patient 34. For purposes of this disclosure, the term fluid should be defined to include any substance that continually deforms or flows under an applied shear stress such as, for example, a liquid or a gas. The flow sensor 56 is configured to measure the flow rate of a fluid passing through the inspiratory limb 40, and to transfer measurement data to the controller 24 (shown in Figure 1). The flow sensor 56 may comprise known technology and therefore will not be described in detail.

The expiratory limb 42 comprises one or more tubes configured to direct exhalation gas from the patient 34. The exhalation gas from the patient 34 can be passed through the CO2 absorber 50 to produce recycled exhalation gas that is transferable back to the patient 34 for rebreathing. Alternatively, some or all of the exhalation gas from the patient 34 can be vented to atmosphere or passed through a hospital scavenging system. The expiratory limb 42 may include a flow sensor 58, a one-way valve 62, and an adjustable pressure limit (APL) valve 64.

The flow sensor 58 is configured to measure the flow rate of a fluid passing through the expiratory limb 42, and to transfer measurement data to the controller 24 (shown in Figure 1). The one-way valve 62 is adapted to regulate fluid flow through the expiratory limb 42 such that fluid is only transferable in a direction away from the patient 34. The APL valve 64 is adapted to set an upper pressure limit within the pneumatic circuit 30.

The endotracheal tube 45 is adapted for insertion into the patient's airway 47. A properly placed endotracheal tube 45 engages and forms a seal with the patient's trachea 49. This seal establishes a generally airtight pneumatic coupling between the patient's lungs 51, the inspiratory limb 40 and the expiratory limb 42. An improperly placed endotracheal tube 45 that fails to seal with the trachea 49 can produce a pneumatic leak. Pneumatic leaks attributable to improper insertion of the endotracheal tube 45 are often difficult to identify; they can cause the endotracheal tube 45 to dislodge; and they interfere with the efficient transfer of breathing gas to the patient.

Referring to Figures 1 and 2, the anesthesia system 8 is adapted to implement controller 24 to automatically identify pneumatic leaks attributable to improper insertion of the endotracheal tube 45. It should be appreciated that the anesthesia system 8 is shown in accordance with an embodiment for illustrative purposes, and that alternate embodiments may implement other devices such as a ventilator system comprising the controller 24 to automatically identify pneumatic leaks attributable to improper insertion of the endotracheal tube 45.

According to one embodiment, the controller 24 may be configured to identify pneumatic leaks based on feedback from the flow sensor 56 and 58. Identified pneumatic leaks may be conveyed to a physician to facilitate the proper insertion of the endotracheal tube 45 during the course of a tracheal intubation. For example, a physician can rely on feedback from the controller 24 to assess the integrity of the pneumatic coupling during the course of a tracheal intubation, and to adjust the position of the endotracheal tube 45 until a minimally acceptable leak is achieved.

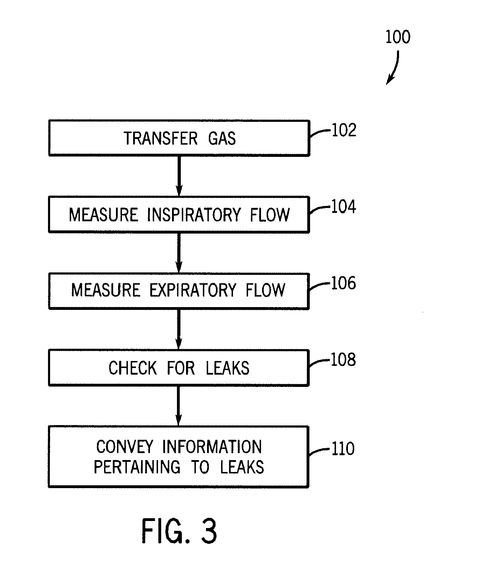

Referring to Figure 3, a flow chart illustrating an algorithm 100 is shown in accordance with an embodiment. The technical effect of the algorithm 100 is to provide user feedback adapted to facilitate the proper insertion of the endotracheal tube 45 during the course of a tracheal intubation. According to one embodiment, the at least a portion of the algorithm 100 comprises a computer program stored on a computer-readable storage medium. The individual blocks 102-110 represent steps that can be performed by the computer 24 (shown in Figure 1).

At step 102, a selectable volume of fresh gas is transferred through the fresh gas inlet 52. In some embodiments it may be advantageous to reconnect the fresh gas inlet 52 downstream relative to the one-way valve 54 such that delivered gas is not diverted away from the patient 34 through the CO2 absorber 50. The fresh gas from the fresh gas inlet 52 passes through the inspiratory limb 40, through the endotracheal tube 45, into the patient's lungs 47, back through the endotracheal tube 45, and through the expiratory limb 42.

At step 104, the controller 24 may measure inspiratory gas flow based on feedback from the flow sensor 56. At step 106, the controller 24 may measure expiratory gas flow based on feedback from the flow sensor 58.

At step 108, the controller 24 may automatically check for pneumatic leaks. According to one embodiment, the controller 24 checks for pneumatic leaks by comparing inspiratory gas flow with expiratory gas flow. As an example, a pneumatic leak may be indicated when measured inspiratory gas flow exceeds expiratory gas flow by a selectable margin. The selectable margin may be implemented to account for minor system losses and thereby reduce false alarms.

At step 110, the controller 24 may convey identified leaks to the user in a known manner such as, for example, one or more alphanumeric symbols, a warning light, an audible alarm, etc. According to one embodiment, the controller is configured to convey identified leaks to the user with a numeric representation indicating the difference between inspiratory flow and expiratory flow displayed on the monitor 25. As previously described, the information conveyed at step 110 may be implemented by a physician to facilitate the proper insertion of the endotracheal tube 45 during the course of a tracheal intubation.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. An anesthesia system (8) comprising:
a pneumatic circuit (30) comprising:
an inspiratory limb (40);
an expiratory limb (42); and
an endotracheal tube (45) configured to form a pneumatic coupling between a patient (34), the inspiratory limb (40) and the expiratory limb (42); and
a controller (24) connected to the pneumatic circuit (30), said controller (24) configured to identify the presence of a leak in the pneumatic coupling.

2. The system (8) of claim 1, further comprising an anesthesia machine (10) comprising the controller (24) connected to the pneumatic circuit (30).

3. The anesthesia system (8) of claim 2, wherein the pneumatic circuit (30) further comprises a first sensor (56) in pneumatic communication with the inspiratory limb (40), and a second sensor (58) in pneumatic communication with the expiratory limb (42).

4. The anesthesia system (8) of claim 3, wherein the controller (24) is configured to identify the presence of a leak in the pneumatic coupling based on feedback from the first sensor (56) and the second sensor (58).

5. The anesthesia system (8) of claim 2, wherein the controller (24) is configured to transfer a gas (29) into the pneumatic circuit (30), implement the first sensor (56) to measure the inspiratory flow rate of the gas (29) through the inspiratory limb (40), and implement the second sensor (58) to measure the expiratory flow rate of the gas (29) through the expiratory limb (42).

6. The anesthesia system (8) of claim 5, wherein the controller (24) is configured to identify the presence of a leak in the pneumatic coupling by comparing the inspiratory flow rate with the expiratory flow rate.

7. The anesthesia system (8) of claim 2, wherein the controller (24) is configured to convey feedback pertaining to the leak in the pneumatic coupling.

8. An anesthesia system (8) comprising:
a pneumatic circuit (30) comprising:
an inspiratory limb (40) adapted to deliver an inspiratory gas to a patient (34);
an expiratory limb (42) adapted to deliver an expiratory gas from the patient (34);
an endotracheal tube (45) configured to form a pneumatic coupling between the patient (34), the inspiratory limb (40) and the expiratory limb (42);
a first sensor (56) in pneumatic communication with the inspiratory limb (40); and
a second sensor (58) in pneumatic communication with the expiratory limb (42); and
an anesthesia machine (10) connected to the pneumatic circuit (30), said anesthesia machine (10) comprising a controller (24) connected to the first (56) and second sensors (58), said controller (24) configured to:
transfer a gas (29) into the inspiratory limb (40) of the pneumatic circuit (30); and
identify the presence of a leak in the pneumatic coupling attributable to an improperly placed endotracheal tube (45) based on feedback from the first (56) and second (58) sensors.

9. The anesthesia system (8) of claim 8, wherein the first sensor (56) is a flow sensor adapted to measure an inspiratory flow through the inspiratory limb (40), and the second sensor (58) is a flow sensor adapted to measure an expiratory flow through the expiratory limb (42).

10. The anesthesia system (8) of claim 9, wherein the controller (24) is configured to compare the inspiratory flow with the expiratory flow to identify the presence of the leak in the pneumatic coupling.

11. The anesthesia system (8) of claim 8, wherein the controller (24) is configured to convey feedback pertaining to the leak in the pneumatic coupling.

12. A method comprising:
transferring a gas into a pneumatic circuit (30);
measuring an inspiratory flow rate of the gas through an inspiratory limb (40) of the pneumatic circuit (30);
measuring an expiratory flow rate of the gas (29) through an expiratory limb (42) of the pneumatic circuit; and
implementing a controller (24) to automatically identify the presence of a pneumatic leak attributable to an improperly placed endotracheal tube (45) based on the inspiratory flow rate and the expiratory flow rate.

13. The method of claim 12, further comprising conveying visual and/or audible feedback pertaining to the pneumatic leak.

14. The method of claim 12 or claim 13, further comprising implementing the visual and/or audible feedback pertaining to the pneumatic leak to more precisely position the endotracheal tube (45).
